# EUROPEAN PATENT SPECIFICATION

(11) **EP 4 593 796 B1**
(45) Date of publication and mention of the grant of the patent: **21.10.2026**
(21) Application number: 23768301.6
(22) Date of filing: 08.09.2023
(51) Int. Cl.: A61K 9/00, A61K 9/20, A61K 31/197, A61P 25/00, A61P 25/02

(54) **VETERINARY COMPOSITIONS FOR USE IN THE TREATMENT OF NEUROPATHIC PAIN**
VETERINÄRE ZUSAMMENSETZUNGEN ZUR VERWENDUNG BEI DER BEHANDLUNG VON NEUROPATHISCHEM SCHMERZ
COMPOSITIONS VÉTÉRINAIRES POUR UTILISATION DANS LE TRAITEMENT DE LA DOULEUR NEUROPATHIQUE

(30) Priority: 29.09.2022 EP 22198759
(43) Date of publication of application: 06.08.2025
(60) Divisional application: 26205713.6
(73) Proprietor: Triviumvet Designated Activity Company, Waterford X91 H5FE (IE)
(72) Inventor: FITZGERALD, Stuart, Tramore County Waterford (IE); BRENNAN, Tom, Clonmel County Tipperary (IE); GRUBB, Louise, Tramore County Waterford (IE); BYRNE, Liam, Dunmore East County Waterford (IE)
(74) Representative: Weldon O'Brien Ltd.
(86) International application number: PCT/EP2023/074798
(87) International publication number: WO 2024/068242

(56) References cited:
- WO-A1-2017/064192
- WO-A1-2022/049149
- WO-A2-2014/119984
- US-A1- 2021 113 515
- SANCHIS-MORA S ET AL: "Pregabalin for the treatment of syringomyelia-associated neuropathic pain in dogs: A randomised, placebo-controlled, double-masked clinical trial", VETERINARY JOURNAL, BAILLIERE TINDALL, LONDON, GB, vol. 250, 5 July 2019 (2019-07-05), pages 55 - 62, XP085757936, ISSN: 1090-0233, [retrieved on 20190705], DOI: 10.1016/J.TVJL.2019.06.006

## Description

### Introduction

Chiari malformation is the collective term given to anatomical anomalies causing herniation of cerebellar tissue through the foramen magnum, which may lead to neurological symptoms. Chiari malformation (CM) is used to describe the condition in humans, while Chiari-like malformation (CLM) is used to describe the canine form. The latter is distinguished from the condition in humans by differences in cerebellar anatomy; however, the etiology and secondary consequences are similar across both species. Chiari malformation is tightly connected to the development of syringomyelia (SM), characterized by the presence of syringes/syrinxes (fluid-filled cavities) within the spinal cord parenchyma. Diagnosis of C(L)M in both human and canine patients has increased due to the greater availability of MRI. The rising popularity of predisposed brachycephalic dog breeds is another factor contributing to an apparent increase in incidence in canine patients.

Neuropathic pain is common in people with chronic neurologic and musculoskeletal diseases such as CM/SM, yet it remains an underappreciated morbidity in veterinary patients. This is likely because assessment of neuropathic pain in people relies heavily on self-reporting, something canine CLM/SM patients are not able to do. In many cases, the presence of neuropathic pain is merely presumed based on the history of the disease and on specific pain behaviours. However, the major concern for canine patients with CLM/SM is that neuropathic pain appears to be more severe than other types of chronic pain, and human neuropathic pain patients reported greater pain, more intense and long-lasting pain, less relief from analgesic drugs, and a greater impairment of quality of life compared with patients with other types of chronic pain. Patients suffering CM/SM have extremely elevated rates of depression, anxiety, and suicide ideation in comparison with non-sufferers. Neuropathic pain can be excruciating and is described by human patients as having burning, stabbing, or shooting qualities with unpleasant tingling, crawling, or electrical sensations. Thus, neuropathic pain can present clinically as a combination of physical signs local to the site of neuropathy (e.g., pain on palpation) and arising from noxious nerve signals local or distal to the site (e.g., scratching), as well as psychiatric disorders. It is unfortunately not possible to be certain whether veterinary patients experience these pain sensations, but odd behaviours associated with presumed causes of neuropathic pain suggest that they are. In fact, behavioural descriptors are an important part of the diagnosis of this type of pain and can be the only clue that neuropathic pain is present in some veterinary patients. However, a "typical" veterinary neuropathic pain patient presents with a combination of behavioural/psychological symptoms and stereotypic, paroxysmal, and often bizarre physical signs. It is possible that disease-specific combinations of descriptors for the latter, and a common panel of descriptors for the former, can characterise the severity of neuropathic pain in individual animals, and may lead to improved outcomes by aiding both (a) case management and therapeutic decisions by veterinarians and (b) new animal drug development by acting as clinical endpoints in trials.

In the canine population, CLM and SM primarily affect small and toy breed dogs. Due to the complexity and variability in clinical signs associated with CLM/SM, and the requirement for an MRI scan for definitive diagnosis, the incidence of canine CLM/SM is not known. However, up to 15% of Cavalier King Charles Spaniels (CKCS) are reported to be symptomatic for the disorder (Thoefner et al., 2020). The prevalence is particularly high among CKCS and increases with age. Analysis of MRI images from 555 clinically unaffected CKCS revealed evidence of SM in 25% of dogs <12 months old, increasing to 70% in dogs >6 years old. Overall, SM was identified in 255 of 555 dogs (~46% prevalence) (Parker et al., 2011).

There is currently a deficit in the ability to adequately assess suffering in canine CLM/SM patients due to the lack of appropriate instruments demonstrated to be sensitive and responsive to disease and treatment effects. Veterinarians must rely on owner observations and physical examination findings to assess the efficacy of treatments, and frequently a mismatch is present in these observations. This condition arises from a congenital malformation that results in a relatively small caudal fossa with respect to the brain causing crowding of the cerebellum and brainstem. Many CKCS with CLM also develop syrinxes within the spinal cord (syringomyelia, SM) because of disruption of cerebrospinal fluid (CSF) flow. Commonly, owners of affected CKCS report signs of phantom scratching, crying out in pain, rubbing of the face and ears, pain on defecation, reluctance to play, collar sensitivity, and aversion to being touched on the head, and these signs are thought to result from SM disrupting sensory pathways.

Pain and sensory deficits in human CM patients have been quantified using thermal and mechanical sensory testing, and patient drawn pain maps. Thermohypoesthesia and decreased sensory perception are common findings in people with CM and SM, but the presence of pain in human patients clearly indicates that sensory changes can involve both gain as well as loss of function. Although the majority of outcome assessments in people with neuropathic pain rely on the patient's description of sensations based on questionnaires or phone call follow-ups, the use of quantitative sensory testing (QST; thermal and mechanical) has been validated using test-retest and interobserver reliability. Furthermore, the duration of sensory deficits quantified by QST before surgery is the best predictor of surgical outcome in human patients with SM (Attal et al., 2004). Treatment options for dogs with CLM/SM focus on pain management, controlling CSF production, and surgical decompression of the caudal fossa. Frequently, these options fail to completely alleviate signs of scratching and pain in these dogs. A major challenge in determining whether treatment options are successful is the difficulty in documenting and quantifying neuropathic pain in these dogs.

Veterinary patients with CLM/SM may display certain clinical signs either visible on clinical examination or reported by owners during history taking. Pet owners may not realize their pet is displaying signs of pain, so veterinarians must question carefully for potential signs in patients at risk. Obvious manifestations may include altered reaction to touch, vocalization in the absence of an overt painful stimulus, phantom scratching, excessive licking or self-mutilation, and persistent lameness/diminished weightbearing on a limb. More subtle signs may include decreased general activity level, reluctance to climb or descend stairs, diminished jumping behaviour, difficulty rising from a seating position, trouble getting into and out of the car, changes in body posture, and altered demeanour or appetite.

Sparks et al. (2018) attempted to develop a tool to capture owner-reported clinical signs for use in clinical trials and to compare owner-reported signs with the presence of pain on neurologic examination and SM on magnetic resonance imaging (MRI). Owners completed a questionnaire and pain/scratch map. Each dog underwent a neurologic examination and craniocervical MRI. Questionnaire responses were developed into scores, area of shading for pain/scratch maps was measured, and consistency of responses between these tools was assessed.

Quantitative sensory testing has been used extensively in human CM patients and the results display a complicated mix of paresthesias, anesthesia, and allodynia. In 1 study, CM patients described an increase in number and area of painful sites on pain drawings but also experienced thermohypoesthesia of the face and body (Thimineur et al. 2002). An important distinction regarding the use of QST in people and dogs is the interpretation of the response. In humans, detection thresholds for the stimulus and the pain threshold for the same stimulus can be differentiated clearly by the subject. This is much more difficult to do in dogs. It may be the case that some dogs show decreased sensitivity whereas others show increased sensitivity or perhaps within a dog there may be bidirectional sensory changes (thermohypoesthesia and mechanical hyperalgesia) as seen in human medical literature. Indeed, the difficulty in interpreting behavioural responses both makes it challenging to understand the pathophysiology of the disease and to assess the efficacy of therapies for this disease. Commonly, von Frey filaments have been used in humans with SM and in various studies of pain in dogs. However, a lack of association between normal dogs and dogs with chronic pain also has been noted using von Frey techniques.

Sparks et al. (2018) found that owner-reported findings did not correlate well with neurologic examination findings and presence and severity of SM on MRI. Extent of shaded areas on maps correlated weakly with questionnaire scores for pain (r2 = 0.213, P = 0.006) and scratch (r2 = 0.104, P = 0.089). Owner-reported findings were not significantly associated with presence or severity of SM or neurologic examination findings. Of 33 symptomatic dogs, only 23 were declared by the owners to experience pain. The median pain score for these painful dogs on a scale of 0-20, incorporating frequency and severity of pain, was 4. Given the many severe impacts on QOL described by human CM/SM patients, this attempt to associate owner-reported signs with disease severity highlighted the deficit in interpretability of CLM/SM severity in this species. This deficit hinders disease recognition by owners and veterinarians as well as impairing assessment of treatment effectiveness or disease progression.

Based on self-reported responses to treatment in human patients and expert opinion in veterinary practice, pregabalin at doses of 5-10 mg/kg twice daily is the preferred medication for CLM/SM-afflicted dogs. Less optimally, gabapentin may be dosed at 10-20 mg/kg three times daily; however, three-times daily dosing is difficult for many dog owners to implement, leading to inadequate control of patient-experienced symptoms. The effectiveness of pregabalin in alleviating symptoms in CLM/SM dogs has been demonstrated in randomized controlled trials, whereas such evidence is currently lacking for gabapentin. Despite the reported effectiveness of pregabalin in dogs with CLM/SM, Sanchis-Mora et al. could only document a moderate effect size of 0.45 in a cohort of CKCS symptomatic for CLM/SM in a crossover placebo-controlled pregabalin field study. These authors employed a specific numerical rating scale (NRS), reporting mean scores of 3.17 (±SD 2.3 units) during treatment, compared with 4.24 (±SD 2.4 units) during the placebo phase. Critically, Sanchis-Mora and colleagues used a liquid formulation of pregabalin approved for use in humans; other veterinary researchers have used hard capsules to administer pregabalin to dogs. As reported by Rusbridge (2019), and many other researchers and veterinary practitioners, aversion to touch is present in many CLM/SM dogs as a result of their severe cranial and cervical neuropathic pain. The severity of pain can also result in a "change in emotional state... becoming more timid, anxious, withdrawn, or aggressive." The requirement for owners to dose such animals with liquid or capsule formulations therefore places a strain on the human-animal bond, as manipulating the dog's head and mouth to administer treatment is invariably uncomfortable for the animal, and places the owner at risk of being bitten as the dog responds to pain and discomfort. Taste masking of liquid formulations for the human palate does not account for canine preferences, and such formulations may therefore be challenging to administer. In the case of capsules or unflavoured tablets, the bitterness of the active ingredient will prevent the dog from consuming the dose should the animal chew the product, as often occurs with oral dosing of companion animals. If the product is an extended-release dose form, chewing presents a toxicity risk for the patient, as disruption of the dose unit may result in acute overdosage of the drug. On the other hand, failure to administer a full therapeutic dose to a CLM/SM dog on a given occasion makes subsequent dosing even more challenging because of learned aversion, greater pain on manipulation for dosing, or both, presenting major concerns for animal welfare.

Inherent variability, insensitivity, and lack of responsiveness in the tools currently available to assess disease severity and treatment effectiveness in CLM/SM hinder the ability of owners and veterinarians to adequately assess and address this painful problem in dogs. Furthermore, these same issues render it hugely challenging to develop and validate treatments for this condition. Global pharmaceutical and medical device regulators require methodologically sound, robust, and repeatable evidence of effectiveness in order to approve interventions for therapeutic use.

In a human study of 27 adults, Cohodarevic et al., (2000) found that pain is an early symptom of SM that can be aggravated by sneezing or coughing. Moreover, the presence of pain was found as the initial symptom in 59% of patients surveyed by the Canadian Syringomyelia Network in 1996, with motor and/or sensory abnormalities identified in 30% of participants, and a combination of pain and sensory/motor symptoms identified in 11% (Cohdarevic et al., 2000). Several human studies have identified a strong correlation between psychological illness and CM. Overall, high rates of moderate to severe depression and anxiety (44% and 60%, respectively) are reported (Garcia et al., 2019). An additional finding of the Canadian Syringomyelia Network survey was that in nearly all (90%) of patients with painful SM there was a connection between stress, activity, and pain, while 47% of participants were completely unable to work due to intractable pain (Cohodarevic et al., 2000). The link between CM, SM, and neuropathic pain more generally and negative psychological impact is well documented. Patients with SM commonly have symptoms of anxiety, memory impairment, and depression (Mueller & Oró, 2013). Descalzi et al. (2017) describe similar evidence in a rodent model determining that chronic neuropathic pain results in adaptations in several brain networks involved in mood, motivation, and reward that are induced by changes in gene expression. A negative effect of neuropathic pain has also been reported in other chronic health conditions in humans, associated with worse quality of life (QOL), greater psychological distress, increased interference with sleep, and loss of more workdays than chronic pain without a neuropathic component.

Canine CLM/SM appears to have both physical and psychological manifestations with resulting deleterious effects on QOL. CLM/SM can have a debilitating effect on a dog's QOL and disrupt the human-animal bond. CLM/SM may lead affected dogs to avoid being petted/groomed, decrease their social interactions, and display aggression towards other dogs and people, including the owner. A myriad of negative impacts of SM on QOL have been described; for example, compulsive phantom scratching induced by the wearing of a collar or harness may make leash walking difficult. In CKCS, phantom scratching may be related to the presence of a mid-cervical syrinx with a transverse width greater than 4 mm and/or extension to the region of the superficial dorsal horn in the C3-C6 spinal segments (Nalborczyk et al., 2017). Such lesions might influence activity of the lumbosacral scratching central pattern generator. It has been suggested that phantom scratching in humans with CM or SM may be a manifestation of paresthesia or allodynia. The term allodynia refers to a condition where a stimulus not typically considered painful and not encoded by nociceptors is perceived to be painful by an individual with somatosensory dysfunction. Mechanical hyperalgesia, cold hyperalgesia, and allodynia are also present in dogs presenting with SM (Moore, 2016).

Diagnosis of CM/SM in non-verbal infants and young children is challenging. Similarly, the multitude of symptoms observed in canine CLM/SM patients can be intermittent, non-specific, or overlooked by owners. In dogs, clinical and behavioural signs that can be logically attributed to CLM/SM include spontaneous vocalization, phantom scratching, allodynia, scoliosis, exercise intolerance, collar sensitivity, pain on defecation, sleep disruption, fearfulness, anxiety, and excitability (Rusbridge et al., 2019).

WO2022049149A describes a chewable veterinary composition comprising an active pharmaceutical ingredient and a carbonate compound. The composition may include a meat flavour.

US20210113515A describes a pregabalin formulation with polmacoxib.

WO2017064192 describes a pregabalin formulation including a lubricant and a filler.

WO2014/119984A describes a biphasic delivery system.

Sanchis-Mora et al Veterinary Journal. https://doi.org/10.1016/j.tvjl.2019.06.006 - 2019 decribes a clinical trial of pregabalin for the treatment of syringomyelia-associated neuropathic pain in dogs.

### Summary of the Disclosure

A veterinary tablet immediate release formulation is described which comprises:
about 25% by weight of pregabalin;
from 5% to 15% by weight of a meat flavour;
from 58% to 69% by weight of microcrystalline cellulose; and
from 1% to 2% by weight of a lubricant such as magnesium stearate.

Also described is a veterinary tablet immediate release formulation consisting essentially of:
about 25% by weight of pregabalin;
from 5% to 15% by weight of a meat flavour;
from 58% to 69% by weight of microcrystalline cellulose; and
from 1% to 2% by weight of a lubricant such as magnesium stearate.

A veterinary tablet immediate release formulation is described which comprises:
about 25% by weight of pregabalin;
about 10% by weight of a meat flavour;
about 64% by weight of microcrystalline cellulose; and
about 1% by weight of magnesium stearate.

Also described is a veterinary tablet immediate release formulation consisting essentially of :
about 25% by weight of pregabalin;
about 10% by weight of a meat flavour;
about 64% by weight of microcrystalline cellulose; and
about 1% by weight of magnesium stearate.

A veterinary tablet immediate release formulation is described which consists of:
about 25% by weight of pregabalin;
about 10% by weight of a meat flavour;
about 64% by weight of microcrystalline cellulose; and
about 1% by weight of magnesium stearate.

Microcrystalline cellulose having a moisture content of less than 1.5% is particularly preferred for enhanced formulation properties.

The term meat flavour as used in this specification includes natural or artificial flavours of beef, pork, chicken, fish, poultry and the like. An example is PC-0125 Artificial Powdered Beef Flavour which contains no ingredients of bovine origin. It is available from Pharma Chemie, Inc., 1877 Midland Street., Syracuse. Nebraska 48666.

Further described is a tablet formulation as defined for use in treating neuropathic pain in a non-human animal. In some cases the non-human animal is a companion animal, such as a canine.

In one case the animal is a Cavalier King Charles Spaniel.

We describe a veterinary tablet immediate release formulation consisting of or consisting essentially of:
about 25% by weight of a gabapentinoid;
from 5% to 15% by weight of a meat flavour;
from 58% to 69% by weight of microcrystalline cellulose having a moisture content of less than 1.5% w/w; and
from 1% to 2% by weight of a lubricant.

The lubricant may be magnesium stearate.

The gabapentinoid is pregabalin.

Also described is a veterinary tablet immediate release formulation consisting essentially of:
about 25% by weight of pregabalin;
about 10% by weight of a meat flavour;
about 64% by weight of microcrystalline cellulose having a moisture content of less than 1.5%; and
about 1% by weight of magnesium stearate.

The tablet formulation provides immediate release in vivo. A tablet formulation is preferable given the challenges of administering a capsule or a liquid formulation to the patients taking the product. The meat flavour assists with patient compliance which is especially important in view of the bitterness of the API. The tablet also has good flow, compaction, disintegration and dissolution properties and is stable over time. The tablet is readily formulated using standard pharmaceutical manufacturing equipment and lends itself well to routine full scale commercial manufacture.

We also describe a medical use of treating a non-human animal having neuropathic pain, the method comprising determining a level of behaviours of the animal and treating the determined neuropathic pain in the non-human animal.

The behaviours may involve at least two, at least three, at least four or all of scratching (displaying visible signs of irritation), anxiousness (displaying nervousness or worry), sensitivity (easily upset or hurt), uncomfortableness (displaying sings of physical discomfort), and restlessness (unable to relax).

The method in one case uses a questionnaire to determine the level of severity of neuropathic pain, analysing the answers to the questionnaire, generating a treatment regimen dependent on the analysis, and administering a gabapentinoid to the non-human animal in the generated treatment regimen. The questionnaire used in the invention is hereinafter referred to as the Questionnaire.

The non-human animal having neuropathic pain is in one case treated by administering a tablet as defined above.

In some cases, the non-human animal is a companion animal, such as a canine, for example a Cavalier King Charles Spaniel.

The method is advantageous in allowing accurate and safe dosing of an effective treatment of a canine patient with neuropathic pain, especially related to CLM/SM, in proportion to the severity of clinical signs displayed by the animal. The Questionnaire score allows caregivers to sensitively and reliably quantify disease severity in an affected animal, and to evaluate effectiveness of pregabalin or other gabapentinoid at the dose currently being administered. The construct of the questionnaire is such as to capture both physical and psychological symptoms of disease, and effects of pregabalin or gabapentinoid treatment on both.

We also describe method for diagnosing neuropathic pain in a non-human animal comprising determining the level of Ease in the animal which may be determined from the behaviour of the animal.

The behaviour may involve at least two, at least three, at least four or all of scratching, anxiousness, sensitivity, uncomfortableness, and restlessness.

The method in one case uses a questionnaire to determine the level of severity of neuropathic pain, analysing the answers to the questionnaire, generating a treatment regimen dependent on the analysis, and administering a gabapentinoid to the non-human animal in the generated treatment regimen.

In some cases, the non-human animal is a companion animal, such as a canine, for example a Cavalier King Charles Spaniel.

The diagnostic method provides a novel means of assessing both physical and psychological aspects of disease in the animal and is sensitive to disease presence and severity. The method has greater sensitivity and reliability than methods currently available.

We also describe a method of monitoring the treatment a non-human animal having neuropathic pain, the method comprising determining a level of behaviours of the animal and monitoring the determined neuropathic pain in the non-human animal. The treatment regimen may be adjusted accordingly.

It is essential to ensure the purity of the final formulation. This was a very significant challenge in this case because of the potential presence of a known impurity of pregabalin which is known as Impurity A and the meat flavour which is present in the formulation. Both the impurity and the meat flavour are amino acids and we also describe an analytical method which we developed which would distinguish between the impurity and the meat flavour.

### Brief Description of the Figures

The invention will be more clearly understood from the following description thereof given by way of example only with reference to the accompanying Figures in which:
Fig 1 is a sample eigenvalue plot from a dataset;
Fig. 2 shows distribution curves of Vitality scores for healthy and CLM/SM dogs; and
Fig. 3 shows distribution curves of Ease scores for healthy and CLM/SM dogs.

### Detailed Description

The range of symptoms displayed by canine CLM/SM patients are wide and potentially confusing. We screened a large number of items, and to eventually select over 30 items for inclusion in initial trials. The items selected were intended to capture constructs such as "vitality," "comfort," "irritation," and "pain/distress," all of which are self-reported by human CM/SM patients to be negatively impacted by their disorder.

In an iterative process, approximately 150 dog owners - 100 healthy dogs and 50 CLM/SM dogs - were invited to complete developmental versions of the assessment instrument. Questions were framed as *"Please think about your dog's behaviour over the past week and use the scale to indicate how much he*/*she is... [descriptor]."* A 7-point Likert scale was presented for each question, with 0 representing "not at all" and 6 being "all the time." For owners of CLM/SM dogs, information was also requested on current treatment regimen.

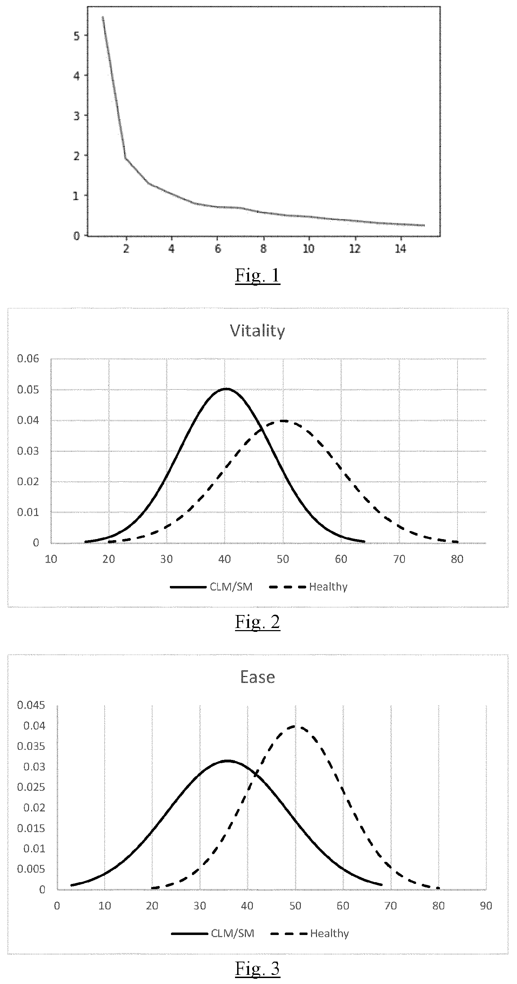

The data gathered were cleaned and analyzed using the sklearn.decomposition FactorAnalysis package in Python. Eigenvalue plots from multiple iterations of the questionnaire data consistently indicated the presence of two major latent variables, accounting for the bulk of the variability present and representing the greatest opportunity to identify deviations from "normal." A sample eigenvalue plot from the final dataset is presented in Fig. 1.

Employing varimax rotation, factor analysis was performed, specifying alternately the presence of 2 and 3 factors. It was apparent that the third factor when present captured descriptors associated with overt pain; e.g. "sore," "limping," "yelping", etc. However, the factor loadings for this factor tended to indicate that two to three parameters would account for >90% of the factor score. Examination of the raw data indicated a low success rate in identifying pain in the sampled dogs using this approach. Moreover, the eigenvalue for this factor was <1, as is evident in the plot above, justifying the exclusion of this latent variable from further investigation.

Of the two factors identified in this analysis, one lent itself to the description *Vitality* on the basis of its constituent parameters; the other was termed *Ease.* Through the several iterations of the questionnaire, descriptors with high correlative values, and those found to have non-significant values between the healthy and diseased groups, were eliminated, leaving the following parameters (major elements with associated factor loadings):

**Table 1**

| **Vitality** | |
|---|---|
| Energetic | 0.72 |
| Lethargic | 0.59 |
| Relaxed | 0.53 |
| Playful | 0.78 |

**Table 2**

| **Ease** | |
|---|---|
| Scratching | 0.58 |
| Anxious | 0.72 |
| Unhappy | 0.71 |
| Sensitive | 0.8 |
| Uncomfortable | 0.78 |
| Restless | 0.33 |

General principles of factor analysis dictate that parameters with loadings >0.3 be retained in the final model. Cronbach's alpha for each of the two factors above indicated a high degree of internal consistency (>0.8 in both cases).

As expected, when sampling a healthy population using bounded outcome measures, the responses from owners of non-CLM/SM dogs exhibit a high degree of skewedness. To facilitate later data manipulation and interpretation, logistic transformation could be applied to the data. Briefly, parameter scores from healthy dogs were converted to values between 0 and 1, applying the formula $Converted score \left(S′\right) = \frac{S + 0.1}{6.2}$

Converted scores obtained were logistically transformed using the formula $S " = log \left(\frac{S ′}{1 - S ′}\right)$

The values obtained were subsequently weighted according to the factor loadings above, and values for each of the two factors, Vitality and Ease, were calculated for each animal. T-scores (with a mean (µ) of 0 and standard deviation (σ) of 1) were calculated for each factor as follows: $T = \frac{S " - μ}{σ}$

To generate intuitive final outputs, T scores were multiplied by 10 and added to 50, providing normalized data with a mean of 50 and standard deviation of 10. Similar transformation was applied to the data from CLM/SM dog owners, employing reference values from the healthy cohort to ensure differentiation of the distribution curves generated as illustrated in Figs 2 and 3.

As can readily be appreciated from Figs 2 and 3, the Ease factor more clearly distinguishes between healthy dogs and those with CLM/SM, indicating its greater sensitivity to the presence of disease. Furthermore, the parameters loading onto the Vitality factor are largely unlikely to be positively impacted by treatments with known effectiveness for the target indication - with the likely exception of "relaxed." Pregabalin, particularly at higher doses, is expected to have a sedative effect, thereby negatively impacting parameters such as "energetic" and "playful." While such effects must of course be documented as adverse drug effects, they must not confound the independent assessment of effectiveness required for an effective benefit-risk assessment.

In contrast, pregabalin, as an anxiolytic and analgesic, may confidently be expected to improve patient scores on such parameters as "anxious," "sensitive," and "uncomfortable." Furthermore, the drug has been demonstrated by Thoefner et al. (2020) to effectively reduce phantom scratching in affected dogs, in the setting of a placebo-controlled treatment trial. Given these facts and the methodical approach taken to this point we have a high degree of confidence that a six-question assessment tool comprising the Ease factor represents a viable endpoint assessment instrument for use in future clinical trials. Notably, the large majority of CLM/SM dogs from which owner responses were gathered in early development of the Questionnaire were receiving treatment with pregabalin or gabapentin when the owners were surveyed; therefore, it was anticipated that the magnitude of difference between healthy dogs and untreated dogs with CLM/SM will be significantly greater. This has indeed been the case in the animals treated at North Carolina State University. However, in-clinic application of the Questionnaire has led us to drop the item "unhappy," as it has been shown to be less responsive to treatment than the remaining 5 items.

### The final content of the Questionnaire is as follows:

*Please think about how your dog has behaved over the past week and use the scale to tell us how well the phrases describe your dog or what he*/*she is doing.*
Scratching (Displaying visible signs of irritation)

| 0: not at all | 1 | 2 | 3 | 4 | 5 | 6: couldn't be more |
|---|---|---|---|---|---|---|
| ○ | ○ | ○ | ○ | ○ | ○ | ○ |

**Anxious** (Displaying nervousness or worry)

| 0: not at all | 1 | 2 | 3 | 4 | 5 | 6: couldn't be more |
|---|---|---|---|---|---|---|
| ○ | ○ | ○ | ○ | ○ | ○ | ○ |

**Sensitive** (Easily upset or hurt)

| 0: not at all | 1 | 2 | 3 | 4 | 5 | 6: couldn't be more |
|---|---|---|---|---|---|---|
| ○ | ○ | ○ | ○ | ○ | ○ | ○ |

**Uncomfortable** (Displaying signs of physical discomfort)

| 0: not at all | 1 | 2 | 3 | 4 | 5 | 6: couldn't be more |
|---|---|---|---|---|---|---|
| ○ | ○ | ○ | ○ | ○ | ○ | ○ |

**Restless** (Unable to relax)

| 0: not at all | 1 | 2 | 3 | 4 | 5 | 6: couldn't be more |
|---|---|---|---|---|---|---|
| ○ | ○ | ○ | ○ | ○ | ○ | ○ |

### Pilot Study and Validation of the Questionnaire

Uniquely, the Questionnaire has now been demonstrated to reliably discriminate between healthy control dogs with no symptoms of CLM/SM, dogs with CLM/SM not currently receiving treatment or being inadequately treated, and the same CLM/SM dogs following appropriate medical management of their symptoms of neurologic pain, under the care of a specialist veterinary neurologist at North Carolina State University. Summary values for untransformed and unweighted scores across the five final questions are presented below.

**Table 3**

| | *n* | Mean | SD |
|---|---|---|---|
| Healthy | 63 | 5.1 | 4.8 |
| Untreated CLM/SM | 8 | 13.5 | 4.4 |
| Treated CLM/SM | 8 | 4.4 | 2.9 |

Using mean and SD values for 63 healthy control dogs as reference data, the untreated CLM/SM dogs have significantly higher CHASE scores, indicating severity of their disease (p < 0.0001 https://www.medcalc.org/calc/comparison_of_means.php). Intervention by a board-certified neurologist and prescription of pregabalin at 5-10 mg/kg twice daily relieved clinical signs and reduced CHASE scores to within the normal range, indicating responsiveness of the questionnaire to standard-of-care medical treatment. Each of the 8 treated dogs (100%) exhibited improvements in symptom severity of at least 5 points on the Questionnaire, equivalent to a large effect size ≥1.04. Using the Questionnaire to solicit owner feedback on disease severity and its impacts on affect and quality of life in this manner, owners and the veterinary neurologist concurred on treatment effects in this 100% of cases. This is to be expected given the potency of pregabalin in the treatment of neuropathic pain; however, no owner assessment tool has ever been reported to detect said effects so sensitively. For example, in a randomized controlled study by Thofner et al. (2020), even the very high dose range of 13 to 19 mg/kg pregabalin yielded concurrence between veterinarian and owner in only 52% of assessments of dogs with CLM/SM.

The unique sensitivity of the Questionnaire to the beneficial effects of gabapentinoid treatment render it essential to the management of causes of neuropathic pain in dogs, including CLM/SM. While "scratching" is also a feature of other causes of neuropathic pain in some animals, for example cervical intervertebral disc disease, it is not as consistent. It is anticipated that other descriptors could replace "scratching" in particular disease settings; for example, "stiff" could be applied to facilitate assessment of the neuropathic component of osteoarthritis; "unsteady" may be particularly applicable to dogs with spondylomyelopathy.

The sensitivity and responsiveness of the questionnaire facilitate appropriate dose titration in the management of such disorders, and Questionnaire scores should be tracked in all patients receiving pregabalin or gabapentin treatment. This is especially the case for interventional clinical research for academic or drug-development purposes, as the Questionnaire may be used as a clinically relevant endpoint reflecting meaningful change in the degree of suffering being experienced by canine patients. Thus, the Questionnaire allows the effectiveness of treatment to be assessed and monitored, and for treatment to be tailored for each animal.

### Example Case: Blinded, Randomized, Controlled Crossover Study to Assess the Safety and Efficacy of Pregabalin in Dogs with CML/SM - "The CHASE Study"

A clinical study is conducted in client-owned dogs with CLM/SM to determine the safety and efficacy profiles of the pregabalin formulation when in clinical use. The study is conducted at specialist veterinary neurology practices and using the CHASE Questionnaire and other evaluations to compare symptom severity at baseline with that during placebo and pregabalin dosing periods. At screening, dogs undergo physical, laboratory, and neurologic examinations as well as craniocervical magnetic resonance imaging and cerebrospinal fluid analysis. Owners complete a global quality of life questionnaire and the CHASE Questionnaire. Based on preliminary data, it is expected that 25% of dogs are positive responders during the placebo period and 75% of dogs are positive responders during the pregabalin period. Using these levels of success, a total of 30 dogs in a crossover study with two treatments and two periods provides adequate power to detect differences between groups at alpha = 0.05.

At least 30 dogs with compatible clinical, historical and imaging findings are enrolled to the study and randomised to one of two study arms: placebo-pregabalin or pregabalin-placebo. During the pregabalin period, dogs are dosed at a rate of 5-10 mg/kg twice daily. Following an initial 2-week dosing period with either placebo or pregabalin, each dog transitions to the alternate treatment in the sequence. Assessments by the specialist veterinarian and the owner are repeated at the end of each treatment period, thereby allowing each dog to act as its own safety and efficacy control. Both the owner and veterinarian remain masked to treatment sequence throughout the study through the use of placebo tablets with similar visual and organoleptic properties.

Pregabalin alleviates pain and clinical signs associated with CLM-SM and this therapeutic effect is captured in Owner-assessed CHASE Questionnaire scores pre- and post-treatment. CHASE Questionnaire scores pre- and post-treatment administration are compared with Owner-assessed quality of life. The efficacy of twice-daily pregabalin in the management of pain and clinical signs is evaluated by comparing changes in total CHASE scores within a subject after treatment with the placebo or pregabalin. In addition to the assessment of the change in total CHASE score, treatment success based on changes in the CHASE score relative to baseline scores are assessed. Success is defined as a ≥5-point improvement (decrease) in the total CHASE score versus baseline, and no single question's score increasing by more than 2 versus baseline. Total questionnaire scores are statistically assessed using methods appropriate for a two period two treatment two sequence crossover design. Specifically, the model includes fixed effects treatment, time and sequence with random dog nested in sequence. Treatment effects are evaluated at alpha = 0.05. Treatment success is similarly evaluated using methods appropriate for binomial data. Alternative methods are employed as appropriate. The percent of dogs receiving rescue treatment is assessed in a similar manner.

Safety is assessed though treatment-emergent adverse events, laboratory results, physical examinations and abnormal observations. All hematology, blood biochemistry, urinalysis, physical examination and adverse event data is summarized using descriptive statistics as appropriate.

### Palatability

In addition to the clinical and other assessments conducted by veterinarians and owners during the CHASE Study outlined above, the palatability of the pregabalin formulation is assessed. Per regulatory guidelines, on each dosing occasion during the study either pregabalin and placebo is offered in an empty bowl or on the ground to assess voluntary acceptance during 30 seconds. In case of failure, the product is offered by hand for an additional 30 seconds by the owner, such that the maximum total offering time is one minute.

The primary endpoint is the overall voluntary acceptance rate which is calculated for the entire period as: $\left(Number of all successful dosings\div Number of all dosings\right) \times 100 %$

The average voluntary acceptance rate is calculated for each time point throughout the treatment period. Changes in the acceptance over time provides information about the overall compliance with the dose regimen, which is of particular interest in case of long term treatment.

For palatability, the overall voluntary acceptance rates reach the threshold of 80% in dogs. Per guidance, this threshold is reached in a group of at least 25 animals in case of multiple administrations. This criterion is met by the enrolment of at least 30 animals to the CHASE study.

### Analytical Challenge.

It is essential to ensure the purity of the final formulation. This was a very significant challenge in this case because of the potential presence of a known impurity of pregabalin which is known as Impurity A and the meat flavour which is present in the formulation. Both the impurity and the meat flavour are amino acids and an analytical method had to be developed which would distinguish between the impurity and the meat flavour.

The development of a stability-indicating reverse phase HPLC method was a very significant technical challenge to overcome. It is a regulatory requirement based on scientific norms to have a method that is capable of separating and detecting all impurities that are expected to be present in the final formulation and to ensure that these are separated from the main drug itself. It is also a requirement to demonstrate specificity i.e. peaks of interest are free from any potential interference from the tablet matrix and to show that the method achieves mass balance (i.e. the sum of impurities under stress conditions approximates any decrease in the main drug concentration). Pregabalin and it's known impurities are classified as amino acids with poor polarity.

Furthermore, as shown in Table 8 below, we found that 10% w/w artificial beef flavour was optimum for the final formulation, but this relatively high concentration means a mixture of a proprietary recipe of likely several hundred flavour compounds would elute very early in a standard reverse phase HPLC column. This presented a significant challenge to separate the beef flavour "fingerprint" from the main drug peak and from the principal impurity of interest, known as Impurity A. In practise, determination of the concentration of Impurity A within the flavour matrix of the tablet was not possible as Impurity A would co-elute with much of the early part of the chromatograms, regardless of solvent system or column type used.

A number of discrimination-based sample preparation approaches, including liquid/liquid extraction and nano ultra-centrifugation filtration were evaluated but were unsuccessful as the polarity of the compounds was too similar to separate. We discovered that HPLC/UV analytics were required to rely on the separation power of the chromatographic column. Several C18 analytical columns were tested and after extensive testing we found that a YMC Triart C18 was most suitable for the separation of Impurity A and the meat flavour. The column is available from YMC, Japan.

Following column selection, the solvent gradient and pH value were fine-tuned in a way that the most abundant placebo peak eluted before Pregabalin and the impurity A peak; noting that the vast majority of the placebo peaks elute in between Pregabalin and impurity A. The detection wavelength was also fine-tuned: several detection wavelengths were considered between 195-215 nm. Finally, the value 200 nm was selected as the optimal sensitive wavelength for determination.

### Tablet formulation

In addition to the major analytical challenge outlined above we encountered great difficulty in developing a tablet formulation of pregabalin for immediate release which was suitable for administration to animals, especially dogs, which had good flow, compaction, and disintegration properties and which was chemically and physically stable over time.

Immediate release means that ≥85% of the labelled amount of drug in the dosage form dissolves within 30 minutes.

### Comparative Example 1

Initially, a series of small-scale trial batches were manufactured using the quantitative formulations presented in the table below. The excipients used were selected based on an extensive literature review of pregabalin drug substance and commercial products. Pregabalin has a bitter taste and known formulations of pregabalin are generally filled into a capsule to avoid compliance issues in view of the known bitterness of pregabalin. However, capsules are not generally suitable for administration to animals; and adding to food presents a compliance challenge due to the bitterness of the pregabalin.

All formulations were prepared by mixing all the ingredients (except magnesium stearate) for five minutes, after which the blend was passed through a 500µm screen and mixed for an additional 15 minutes. Magnesium stearate was added and mixed for five minutes, and this final blend was directly compressed into tablets using a single station tablet press. Round and oblong tooling were both used during compression to determine the impact on tablet quality.

**Table 4: Batch formulation for PGB.210830.01.02, PGB.210830.02.02, PGB.210830.03.02, PGB.210901.01.02 90mg Tablets**

| **Batch number** | **PGB.210830.01.02** | **PGB.210830.02.02** | **PGB.210830.03.02** | **PGB.210901.01.02** |
|---|---|---|---|---|
| **Formulation** | **mg per tablet** | **mg per tablet** | **mg per tablet** | **mg per tablet** |
| Pregabalin | 90 | 90 | 90 | 90 |
| MCC Prosolv HD90 | 90 | - | - | 45 |
| Mannitol | - | 90 | - | 45 |
| Starch 1500 | - | - | 90 | - |
| Sodium Starch Glycolate | 15 | 15 | 20 | 15 |
| Isomalt | 9 | 9 | - | 9 |
| Sucralose | 3 | 3 | 3 | 3 |
| Beef flavour | 90 | 90 | 90 | 90 |
| **Silica** | - | - | 4 | - |
| **Magnesium Stearate** | 3 | 3.6 | 4 | 3 |
| **Total** | **300** | **300.6** | **301** | **300** |
| Batch scale | 100g | 100g | 100g | 50g |

**Table 5: Physical characterisation ofPGB.210830.01.02, PGB.210830.02.02, PGB.210830.03.02, PGB.210901.01.02 90mg Tablets**

| **Analysis** | **PGB.210830.01.02** | **PGB.210830.02.02** | **PGB.210830.03.02** | **PGB.210901.01.02** |
|---|---|---|---|---|
| Hardness (N) | 65 | 50 | 40 | 60 |
| Diameter (mm) | 9 | 9 | 9 | 14x7 |
| Disintegration (min) | 5 | 4 | 4 | 5 |

The formulation containing Starch 1500 exhibited poor compaction properties and tablets had poor hardness. Some sticking and capping issues were observed for tablets produced with oblong tooling.

### Comparative Example 2

In an effort to improve tablet compaction properties, a formulation including MCC PH102 and Silica was introduced, along with a formulation including Starch Startab. Tablet weights were increased to 360mg and 400mg to determine the effect on tablet quality and processing. These larger tablets were compressed with a slightly larger punch (10mm). The batches were manufactured at small scale (between 50g and 100g) and were compressed into tablets using a single station tablet press. The formulation containing Starch Startab exhibited poor compaction properties and tablets had poor hardness. The formulations manufactured with combinations of Microcrystalline Cellulose and Mannitol provided the best physical properties and these formulations were selected for further trials.

### Comparative Example 3

A series of batches were manufactured with the same qualitative formulations and manufacturing process as the previous series of trials. The batch scale was increased to between 300g and 450g to allow for enough tablets to be produced for a preliminary stability study. For the formulation containing Prosolv ^{®} HD90 (High Density grade), this was replaced with Prosolv ^{®} 90 to determine the effect on dissolution. Prosolv ^{®} HD90 has a higher disintegration time than Prosolv ^{®} 90.

**Table 6: Batch formulation for PGB.210913.01.02, PGB.210913.02.02 & PGB.210913.03.02 90mg Tablets**

| **Batch number** | **PGB.210913.01.02** | **PGB.210913.02.02** | **PGB.210913.03.02** |
|---|---|---|---|
| **Formulation** | **mg per tablet** | **mg per tablet** | **mg per tablet** |
| Pregabalin | 90 | 90 | 90 |
| Prosolv HD90 | 45 | - | - |
| SMCC90 | - | 73.2 | - |
| MCC PH102 | - | - | 94.5 |
| Mannitol | 45 | 73.2 | 94.5 |
| Sodium Starch Glycolate | 15 | 18 | 20 |
| Isomalt | 9 | 9 | |
| Sucralose | 3 | 3 | 3 |
| Beef flavour | 90 | 90 | 90 |
| Silica | - | - | 4 |
| Magnesium Stearate | 3 | 3.6 | 4 |
| **Total** | **300** | **360** | **400** |
| Batch scale | 330g | 400g | 450g |

**Table 7: Physical characterisation of PGB.210913.01.02, PGB.210913.02.02 & PGB.210913.03.02 90mg Tablets**

| **Analysis** | **PGB.210913.01.02** | **PGB.210913.02.02** | **PGB.210913.03.02** |
|---|---|---|---|
| Tablet weight (mg) | 295 - 297 | 361 - 363 | 402 - 404 |
| Hardness (N) | 48 - 56 | 45 - 61 | 82 - 96 |
| Diameter (mm) | 9 | 10 | 10 |
| Disintegration (min) | 4 - 5 | 3-4 | 4-5 |

PGB.210913.01.02 and PGB.210913.02.02 both exhibited some sticking and a limited hardness range was achieved. High ejection forces were also observed. PGB.210913.03.02 processed better than the other batches with tablets produced showing less sticking and improved hardness.

The tablets all achieved immediate release of pregabalin - release of >95% in 30 minutes. Therefore, it was decided to place all three formulations on stability in 75ml HDPE containers for 6 months at 25°C/ 60% RH with and without a desiccant, and 40°C/ 75% RH with a desiccant. Surprisingly, we found that the physical appearance of the tablets deteriorated and there was also a significant deterioration in the tablet hardness: a major friability issue. This was also evident from dissolution testing.

The comparative examples above clearly show the great level of difficulty in providing an acceptable veterinary tablet formulation of a pregabalin.

After further research and development, we reduced a number of carbohydrates, reduced the amount of beef flavour and used a low moisture grade microcrystalline cellulose which is the main carrier excipient. The tablets were surprisingly found to be acceptable for veterinary use and to have excellent stability and hardness.

### Example 1 - Preferred Formulation

**Table 8 - 30mg, 90mg & 180mg tablet formulation:**

| **Formulation** | **mg per tablet** | | |
|---|---|---|---|
| Pregabalin | 30.00 | 90.00 | 180.00 |
| Microcrystalline Cellulose (Avicel PH 112) | 76.67 | 230.00 | 460.00 |
| Artificial Powdered Beef Flavour (PC-0125) | 12.00 | 36.00 | 72.00 |
| Magnesium Stearate (Veg. Grade) PhEur/NF | 1.33 | 4.00 | 8.00 |
| Total | 120.0 | 360.0 | 720.0 |

To maximise dosing of different weights of dogs, 3 strengths are provided which are dose proportional and compressed from a common blend.

**Table 9 - 30, 90, and 180 mg tablets:**

| **Test** | **Specification** | | |
|---|---|---|---|
| | 30 mg | 90 mg | 180 mg |
| Appearance | 6 mm round flat face radius edge off-white to light brown flecked tablets, plain on both sides. | 9.5mm Round biconvex off-white to light brown flecked tablets, plain on one side with a break-line on the other side | 13 mm round flat face radius edge off-white to light brown flecked tablets, plain on both sides |
| Average Weight | 120 mg ±5% | 360 mg ±5% | 720 mg ±5% |

Pregabalin API is available from Hetero Drugs Limited, Plot No.1, Hetero Infrastructure SEZ Ltd, N. Narasapuram (Village), Nakkapalli (Mandal), Visakhapatnam (District) - 531 081, Andhra Pradesh, India.

PC-0125 Artificial Powdered Beef Flavour contains no ingredients of bovine origin. It is available from Pet Flavors LLC, 585 Distribution Dr, Suite 4, Melbourne, FL 32904, USA.

Microcrystalline Cellulose (Avicel PH 112) is a directly compressible microcrystalline cellulose grade with a low moisture content (LOD ≤ 1.5% w/w). It is available from Dupont.

Magnesium Stearate is of non-animal origan and meets the requirements of the European and US Pharmacopiea. It is available from Merck KGaA, Darmstadt, Germany.

The formulation was prepared by mixing all ingredients (except magnesium stearate) for five minutes, after which the blend was passed through a 500µm screen and mixed for an additional 15 minutes. Magnesium stearate was added and mixed for five minutes, and this final blend was directly compressed into tablets using a single station tablet press.

The tablets were found to have excellent stability and hardness over time, which aids a consistent rate of drug release in-vivo. This is especially significant as 10% w/w artificial beef flavour was found to be optimum for the final formulation per Table 8 and earlier trials with high flavour concentration showed softening over time which would fail the regulatory requirements for "significant change".

### Stability Data for Preferred Formulations

**Table 10 - stability summary data for sample batch @ 25 °C/60 % RH**

| **Test** | **Specification** | **Initial** | **1 month** | **2 month** | **3 month** | **6 month** | **9 month** | **12 month** |
|---|---|---|---|---|---|---|---|---|
| **Appearance** | Round biconvex off-white to light brown flecked tablets, plain on one side with a break-line on the other side | Complies | Complies | Complies | Complies | Complies | Complies | Complies |
| **Assay** | 90-110% | 101.86 | 101.46 | 103.14 | 101.42 | 98.76 | 101.79 | 101.29 |
| **Related substances** | | | | | | | | |
| Impurity C: | NMT 1.0% | ND | 0.01 | 0.02 | 0.02 | 0.03 | 0.04 | 0.05 |
| Single unknown: | NMT 1.0% | 0.04 | ND | ND | ND | ND | ND | ND |
| Total impurities: | NMT 1.0% | 0.04 | 0.01 | 0.02 | 0.02 | 0.03 | 0.04 | 0.05 |
| **Dissolution** | ≥ 80%(Q) in 30 min | 98.2 | 97.0 | 102.6 | 96.3 | 96.8 | 103.2 | 101.0 |
| **Hardness** | As found (N) | 72 (7.3 kp) | 72 (7.3 kp) | 74 (7.5 kp) | 76 (7.7 kp) | 71 (7.2 kp) | 79 (8.1 kp) | 76 (7.7 kp) |

It will be noted from the table above that the tablets provided consistent dissolution data and excellent hardness over time.

**Table 11: accelerated stability summary data for sample batch @ 40 °C/75 % RH**

| **Test** | **Specification** | **Initial** | **1 month** | **2 months** | **3 months** | **6 months** |
|---|---|---|---|---|---|---|
| **Appearance** | Round biconvex off-white to light brown flecked tablets, plain on one side with a break-line on the other side | Complies | Complies | Complies | Complies | Complies |
| **Assay** | 90 - 110% | 101.86 | 98.41 | 101.02 | 99.60 | 98.53 |
| **Related substances** | | | | | | |
| Impurity C: | NMT 1.0% | ND | 0.01 | 0.06 | 0.09 | 0.20 |
| Single unknown: | NMT 1.0% | 0.04 | ND | ND | ND | ND |
| Total impurities: | NMT 1.0% | 0.04 | 0.01 | 0.06 | 0.09 | 0.20 |
| **Dissolution** | ≥ 80%(Q) in 30 min | 98.2 | 99.0 | 104.8 | 98.7 | 97.2 |
| **Hardness** | As found (N) | 72 (7.3 kp) | 74 (7.5 kp) | 67 (6.8 kp) | 67 (6.8 kp) | 52 (5.3 kp) |

It will be noted from the table above that the tablets provided consistent dissolution data and excellent hardness during accelerated stability testing over time.

| **Test** | **Range of results (40°C/75%RH)** | | | |
|---|---|---|---|---|
| | **30 mg** | | **180 mg** | |
| | **Initial** | **6 Months** | **Initial** | **6 Months** |
| Appearance | Meets test | Meets test | Meets test | Meets test |
| Assay (%w/w, by HPLC) | 97.2-97.9 | 98.0-99.8 | 99.3-101.2 | 100.2-101.1 |
| USP related compound C | <LOQ | <LOQ | <LOQ | <LOQ |
| Unknown Impurities | <LOQ | <LOQ | <LOQ | <LOQ |
| Total | <LOQ | <LOQ | <LOQ | <LOQ |
| Dissolution | 99.4-102.9 | 97.2-98.0 | 98.8-99.5 | 100.5-100.9 |
| TAMC | <10 | <10 | <10 | <10 |
| TYMC | <10 | <10 | <10 | <10 |
| *E. coli* | Absent | Absent | Absent | Absent |

| **Test** | **Range of results (25°C/60%RH)** | | | |
|---|---|---|---|---|
| | **30 mg** | | **180 mg** | |
| | **Initial** | **6 Months** | **Initial** | **6 Months** |
| Appearance | Meets test | Meets test | Meets test | Meets test |
| Assay (%w/w, by HPLC) | 97.2-97.9 | 97.2-99.2 | 99.3-101.2 | 100.5-101.5 |
| USP related compound C | <LOQ | <LOQ | <LOQ | <LOQ |
| Unknown Impurities | <LOQ | <LOQ | <LOQ | <LOQ |
| Total | <LOQ | <LOQ | <LOQ | <LOQ |
| Dissolution | 99.4-102.9 | 95.9-100.0 | 98.8-99.5 | 99.8-100.7 |
| TAMC | <10 | <10 | <10 | <10 |
| TYMC | <10 | <10 | <10 | <10 |
| *E. coli* | Absent | Absent | Absent | Absent |

### Treatment regimens

Following diagnosis of CLM/SM and completion of the Questionnaire by the dog's owner at baseline, treatment should begin immediately.

### Gabapentinoids

Pregabalin is an example of a class of drugs known as gabapentinoids, the therapeutic class of choice for medical management of CLM/SM. Treatment should begin at the lower end of the therapeutic range (e.g., 5 mg/kg twice daily for pregabalin) so as to minimise sedative side effects during the early days of treatment. Gabapentinoids including pregabalin act through selective inhibition of voltage-gated calcium channel α₂δ subunits of the dorsal spinal horn (Moore, 2016). Clinically used gabapentinoids include gabapentin, pregabalin, and mirogabalin, as well as a gabapentin prodrug, gabapentin enacarbil. Additionally, phenibut has been found to act as a gabapentinoid in addition to its action of functioning as a GABAB receptor agonist. Further analogues like imagabalin are in clinical trials but have not yet been approved. Other gabapentinoids which are used in scientific research but have not been approved for medical use include atagabalin, 4-methylpregabalin and PD-217,014. Pregabalin has better potency and binding affinity than gabapentin (Alles et al., 2020). Pregabalin decreases presynaptic calcium currents and in doing so inhibits the release of several neurotransmitters, including glutamate, substance P, calcitonin gene-related peptide, and norepinephrine. Decreases in the activity of these or related "fear circuits" that underlie the pathophysiology of certain anxiety disorders are of particular benefit in the treatment of CLM/SM (Strawn & Geracioti, 2007).

Following an initial period of treatment that may range from several days to several months, the dog's owner should again complete the Questionnaire. Persistently elevated questionnaire scores indicate a requirement to increase the dose towards the higher end of the therapeutic range (10 mg/kg twice daily for pregabalin). Patients with intractable symptoms and persistently elevated CHASE scores may benefit from one or more of the following adjunctive medical therapies, with doses of each adjusted within the specified ranges as appropriate to Questionnaire scores, clinical findings and adverse effects.

### Non-steroidal anti-inflammatory drugs

Inhibit production of prostaglandins through actions on cyclooxygenase (COX) pathway.

NSAIDs can result in vomiting and diarrhoea; however, an animal that has an adverse reaction to one drug will not necessary react to another. Not recommended for animals with kidney disease. Small risk that NSAIDs may precipitate heart failure in animals with pre-existing heart disease.

### Paracetamol (acetaminophen)

Central analgesic effect mediating descending serotonergic pathways either through inhibition of prostaglandin synthesis or through metabolite influencing cannabinoid receptors. This drug has been found to be useful for 'break-through', short-term pain management (i.e., allowing the owner to 'top-up' existing pain relief). Potential for renal, hepatic, gastrointestinal, and haematologic (methemoglobinaemia) adverse effects. High doses can cause keratoconjunctivitis sicca (dry eye). Must be used with caution as dogs do not metabolise it as well as people.

### Topiramate

Antiepileptic drug with multiple possible mechanisms of action, including carbonic anhydrase inhibition. Dosed at 10 mg/kg three times daily. It should be avoided or used with caution in patients with hepatic or renal disease. The most common adverse effect is sedation and ataxia, which is more likely with polypharmacy. Gastrointestinal adverse effects including inappetence/anorexia may be seen. Irritability, aggression, chewing of digits and facial rubbing have been reported.

### N-methyl-d-aspartate (NMDA) antagonists

NMDA antagonists may reduce nociceptive activation as adjunctive therapy (i.e., with another drug such as pregabalin). Amantadine and memantine may be dosed at 3-5 mg/kg once daily and 0.3 to 1 mg/kg twice daily, respectively. The most common adverse effects are sedation and ataxia, which is more likely with polypharmacy. Agitation or gastrointestinal adverse effects may be seen. It should be avoided or used with caution in patients with glaucoma, hepatic disease, renal disease, congestive heart failure, atopic dermatitis or seizure disorders.

### Amitriptyline

This tricyclic antidepressant should be dosed at 0.25 to 2 mg/kg once or twice daily. It acts by blocking re-uptake of serotonin and norepinephrine neurotransmitters. The most common adverse effect is sedation, which is more likely with polypharmacy. Other possible adverse effects include hyperexcitability, seizures, dysrhythmias, bone marrow suppression, diarrhoea, vomiting, hypersalivation. Not advised in animals with seizures or epilepsy. Caution in patients with thyroid disorders, urinary retention, hepatic disorders, keratoconjunctivitis sicca, glaucoma, cardiac rhythm disorders or diabetes.

### Drugs that may reduce the production of cerebrospinal fluid

Omeprazole, an inhibitor of H+/K+-activated ATPase (in the choroid plexus Na+/K+-ATPase regulates the production of CSF), may be dosed at 0.5-1.0 mg/kg once or twice daily. Reported adverse effects include nausea, diarrhoea, constipation and skin rashes. Cimetidine is a histamine H2 receptor antagonist dosed at 5-7 mg/kg PO TID. Adverse effects with this antacid are rare even at high doses. Liver and kidney toxicity have been reported. In people, cimetidine has been reported (rarely) to be associated with headache. Cimetidine may increase the kidney clearance of gabapentin. Acetazolamide, a carbonic anhydrase inhibitor, is dosed at 4-8 mg/kg once daily. Adverse effects are common especially with long-term use and may include anorexia, gastrointestinal signs, bone marrow depression, metabolic derangement (hypokalaemia, hypochloraemia, hyponatraemia, hyperglycaemia), hepatic insufficiency, hypersensitivity reactions (eg, rash) and CNS signs (sedation, depression, weakness, excitement).

### Prednisone/ prednisolone/methylprednisolone

Corticosteroids are dosed initially at 0.5 mg/kg once daily, then decreased to the lowest possible, ideally alternate day, dose that controls signs and maintains CHASE questionnaire scores at a normal level. Neuroactive steroids modulate pain sensitivity and reduce neuropathic pain. Possible effect on aquaporin-4 expression (water channels) in spinal cord. An option for severe SM-S-associated weakness or phantom scratching. However, long-term use is not recommended due to adverse effects. Chronic use results in muscle loss, thereby increasing weakness and animals are often lethargic and heat intolerant; signs which are easily confused with CM-P and SM-S. Other possible adverse effects include: vomiting; diarrhoea; increased urination and drinking and when combined with diuretics can result in potassium depletion; increased appetite and increased calorific intake that results in weight gain; skin changes and poor hair growth; delayed wound healing and increased susceptibility to infection. This drug should not be given to patients that are pregnant, have diabetes mellitus or kidney disease.

### Cannabidiol (CBD oil/hemp extract)

Cannabinoids act via cannabinoid receptors and affect the activities of many other receptors, ion channels and enzymes. They inhibit release of neurotransmitters and neuropeptides from presynaptic nerve endings, modulate postsynaptic neuron excitability, activate descending inhibitory pain pathways and reduce neural inflammation. Cannabinoids appear to be well tolerated in dogs at a dose of 2 mg/kg. Serum biochemistry may show an increase in alkaline phosphatase, presumed due to liver enzyme induction.

### List of References

Alles, S. R. A., Cain, S. M., & Snutch, T. P. (2020). Pregabalin as a Pain Therapeutic: Beyond Calcium Channels. Frontiers in Cellular Neuroscience, 14, 1-9. https://doi.org/10.3389/fncel.2020.00083
Attal, N., Parker, F., Tadié, M., Aghakani, N., & Bouhassira, D. (2004). Effects of surgery on the sensory deficits of syringomyelia and predictors of outcome: a long-term prospective study. Journal of Neurology, Neurosurgery & Psychiatry, 75(7), 1025-1030. Cohodarevic, T., Mailis, A., & Montanera, W. (2000). Syringomyelia : Pain, Sensory Abnormalities, and Neuroimaging. 1(1), 54-66. https://doi.org/https://doi.org/10.1016/S1526-5900(00)90088-9
Descalzi, G., Mitsi, V., Purushothaman, I., Gaspari, S., Avrampou, K., Loh, Y. E., Shen, L., & Zachariou, V. (2017). Neuropathic Pain Promotes Adaptive Changes in Gene Expression in Brain Networks Involved in Stress and Depression. Science Signaling, 10(471). https://doi.org/10.1126/scisignal.aaj1549
Garcia, M., Allen, P., Li, X., & Houston, J. (2019). An Examination of Pain, Disability, and the Psychological Correlates of Chiari Malformation Pre- and Post-Surgical Correction. Disability and Health Journal, 12(4), 649-656. https://doi.org/https://doi.org/10.1016/j.dhjo.2019.05.004 Moore, S. A. (2016). Managing Neuropathic Pain in Dogs. Frontiers in Veterinary Science, 3, 1-8. https://doi.org/10.3389/fvets.2016.00012
Mueller, D. M., & Oró, J. J. (2013). The Chiari Symptom Profile: Development and Validation of a Chiari-/Syringomyelia-Specific Questionnaire. Journal of Neuroscience Nursing, 45(4), 205-210. https://doi.org/10.1097/JNN.0b013e3182986573
Nalborczyk, Z. R., McFadyen, A. K., Jovanovik, J., Tauro, A., Driver, C. J., Fitzpatrick, N., Knower, S. P., & Rusbridge, C. (2017). MRI Characteristics for "Phantom" Scratching in Canine Syringomyelia. BMC Veterinary Research, 13(1), 1-10. https://doi.org/10.1186/s12917-017-1258-2
Parker, J. E., Knowler, S. P., Rusbridge, C., Noorman, E., & Jeffery, N. D. (2011). Prevalence of Asymptomatic Syringomyelia in Cavalier King Charles Spaniels. Veterinary Record, 168(25), 667. https://doi.org/10.1136/vr.d1726
Rusbridge, C., McFadyen, A., & Knower, S, P. (2019). Behavioral and Clinical Signs of Chiari-like Malformation-Associated Pain and Syringomyelia in Cavalier King Charles Spaniels. Journal of Veterinary Internal Medicine, 33(5), 2138-2150. https://doi.org/10.1111/jvim.15552
Sanchis-Mora, S., Chang, Y. M., Abeyesinghe, S. M., Fisher, A., Upton, N., Volk, H. A., & Pelligand, L. (2019). Pregabalin for the treatment of syringomyelia-associated neuropathic pain in dogs: A randomised, placebo-controlled, double-masked clinical trial. Veterinary Journal. https://doi.org/10.1016/j.tvjl.2019.06.006
Sparks, C. R., Cerda-Gonzalez, S., Griffith, E. H., Lascelles, B. D. X., & Olby, N. J. (2018). Questionnaire-based analysis of owner-reported scratching and pain signs in Cavalier King Charles Spaniels screened for Chiari-like malformation and syringomyelia. Journal of veterinary internal medicine, 32(1), 331-339. Strawn, J. R., & Geracioti, T. D. (2007). The Treatment of Generalized Anxiety Disorder with Pregabalin, an Atypical Anxiolytic. Neuropsychiatric Disease and Treatment, 3(2), 237-243. https://doi.org/10.2147/nedt.2007.3.2.237
Thimineur, M., Kitaj, M., Kravitz, E., Kalizewski, T., & Sood, P. (2002). Functional abnormalities of the cervical cord and lower medulla and their effect on pain: observations in chronic pain patients with incidental mild Chiari I malformation and moderate to severe cervical cord compression. The Clinical journal of pain, 18(3), 171-179.Thoefner, M. S., Skovgaard, L. T., McEvoy, F. J., Berendt, M., & Bjerrum, O. J. (2020). Pregabalin Alleviates Clinical Signs of Syringomyelia-related Central Neuropathic Pain in Cavalier King Charles Spaniel Dogs: A Randomized Controlled Trial. Veterinary Anaesthesia and Analgesia, 47(2), 238-248. https://doi.org/10.1016/j.vaa.2019.09.007

## Claims

1. A veterinary tablet immediate release formulation comprising:
about 25% by weight of pregabalin;
from 5% to 15% by weight of a meat flavour;
from 58% to 69% by weight of microcrystalline cellulose; and
from 1% to 2% by weight of a lubricant.

2. A veterinary tablet formulation as claimed in claim 1 wherein the lubricant is magnesium stearate.

3. A veterinary tablet immediate release formulation according to claim 2, comprising:
about 25% by weight of pregabalin;
about 10% by weight of a meat flavour;
about 64% by weight of microcrystalline cellulose; and
about 1% by weight of magnesium stearate.

4. A veterinary tablet formulation as claimed in claim 1 or 3 wherein the microcrystalline cellulose has a moisture content of less than 1.5%.

5. A tablet formulation as claimed in any of claims 1 to 4 for use in treating neuropathic pain in a non-human animal.

6. A tablet formulation for use as claimed in claim 5 wherein the non-human animal is a companion animal.

7. A tablet formulation for use as claimed in claim 6 wherein the companion animal is a canine.

8. A tablet formulation for use as claimed in claim 7 wherein the animal is a Cavalier King Charles Spaniel.

## Patentansprüche

1. Tierärztliche Tablettenformulierung mit sofortiger Freisetzung, die Folgendes umfasst:
etwa 25 Gewichts-% Pregabalin;
von 5 Gewichts-% bis 15 Gewichts-% eines Fleischaromas;
von 58 Gewichts-% bis 69 Gewichts-% mikrokristalline Cellulose; und
von 1 Gewichts-% bis 2 Gewichts-% eines Schmiermittels.

2. Tierärztliche Tablettenformulierung nach Anspruch 1, wobei das Schmiermittel Magnesiumstearat ist.

3. Tierärztliche Tablettenformulierung mit sofortiger Freisetzung nach Anspruch 2, die Folgendes umfasst:
etwa 25 Gewichts-% Pregabalin;
etwa 10 Gewichts-% eines Fleischaromas;
etwa 64 Gewichts-% mikrokristalline Cellulose; und
etwa 1 Gewichts-% Magnesiumstearat.

4. Tierärztliche Tablettenformulierung nach Anspruch 1 oder 3, wobei die mikrokristalline Cellulose einen Feuchtigkeitsgehalt von weniger als 1,5 % aufweist.

5. Tablettenformulierung nach einem der Ansprüche 1 bis 4 für die Verwendung bei der Behandlung von neuropathischem Schmerz bei einem nicht-menschlichen Tier.

6. Tablettenformulierung für die Verwendung nach Anspruch 5, wobei das nichtmenschliche Tier ein Haustier ist.

7. Tablettenformulierung für die Verwendung nach Anspruch 6, wobei das Haustier ein Hund ist.

8. Tablettenformulierung für die Verwendung nach Anspruch 7, wobei das Tier ein Cavalier King Charles Spaniel ist.

## Revendications

1. Formulation vétérinaire sous forme de comprimé à libération immédiate, comprenant :
environ 25% en poids de prégabaline ;
de 5% à 15% en poids d'un arôme de viande ;
de 58% à 69% en poids de cellulose microcristalline ; et
de 1% à 2% en poids d'un lubrifiant.

2. Formulation vétérinaire sous forme de comprimé selon la revendication 1, dans laquelle le lubrifiant est le stéarate de magnésium.

3. Formulation vétérinaire sous forme de comprimé à libération immédiate selon la revendication 2, comprenant :
environ 25% en poids de prégabaline ;
environ 10% en poids d'un arôme de viande ;
environ 64% en poids de cellulose microcristalline ; et
environ 1% en poids de stéarate de magnésium.

4. Formulation vétérinaire sous forme de comprimé selon la revendication 1 ou 3, dans laquelle la cellulose microcristalline présente une teneur en humidité inférieure à 1,5%.

5. Formulation sous forme de comprimé selon l'une quelconque des revendications 1 à 4, pour une utilisation dans le traitement de la douleur neuropathique chez un animal non humain.

6. Formulation sous forme de comprimé pour une utilisation selon la revendication 5, l'animal non humain étant un animal de compagnie.

7. Formulation sous forme de comprimé pour une utilisation selon la revendication 6, l'animal de compagnie étant un canidé.

8. Formulation sous forme de comprimé pour une utilisation selon la revendication 7, l'animal étant un Cavalier King Charles Spaniel.
